Europäisches Patentamt

European Patent Office

Office européen des brevets

(19)

(11) Numéro de publication: **0 213 050**
**B1**

## FASCICULE DE BREVET EUROPEEN

(45) Date de publication du fascicule du brevet: **09.01.91**

(51) Int. Cl.⁵: **A 61 M 1/16**

(21) Numéro de dépôt: **86420209.8**

(22) Date de dépôt: **05.08.86**

(54) **Dispositif de mesure de la quantité d'ultrafiltrat éliminée pendant un traitement de dialyse.**

(30) Priorité: **09.08.85 IT 5369585 u**

(43) Date de publication de la demande:
**04.03.87 Bulletin 87/10**

(45) Mention de la délivrance du brevet:
**09.01.91 Bulletin 91/02**

(84) Etats contractants désignés:
**BE CH DE FR GB IT LI NL SE**

(56) Documents cités:
**FR-A- 765 258**
**FR-A-2 472 936**
**US-A-2 611 269**

(73) Titulaire: **HOSPAL AG**
**Missionsstrasse 60/62**
**CH-4012 Basel (CH)**

(72) Inventeur: **Borsari, Gianni**
**Via Rubadello 29**
**I-41036 Medolla Modena (IT)**

(74) Mandataire: **Gauckler, Jacques**
**HOSPAL C.O.T. SCE Brevets B.P. 21 13, Avenue**
**de Lattre de Tassigny**
**F-69881 Meyzieu Cédex (FR)**

Courier Press, Leamington Spa, England.

EP 0 213 050 B1

**Description**

La présente invention concerne un dispositif de mesure de la quantité d'ultrafiltration éliminée pendant un traitement de dialyse.

Un dispositif de mesure de l'ultrafiltration est décrit dans le brevet FR—A—2 472 936. Selon l'enseignement de ce brevet, le liquide correspondant à l'ultrafiltration du sang s'accumule dans un réservoir, muni d'un capteur de niveau. Lorsque le liquide atteint le capteur, un système de jeux de vannes et de chambres complémentaires associé à une pompe provoque la vidange de ce réservoir.

Un totaliseur est incrémenté à chaque atteinte du niveau du capteur par le liquide s'accumulant dans le réservoir, ce qui permet de totaliser l'ultrafiltration effectuée.

Dans le cas où l'ultrafiltration du sang est effectuée grâce à la mise en oeuvre d'une pompe permettant d'extraire du circuit de liquide de dialyse une quantité de liquide correspondant à la quantité d'ultrafiltrat extraite du sang, la mesure de la quantité d'ultrafiltration est effectuée en disposant un récipient gradué à l'aval de la pompe d'ultrafiltration. Le contrôle de la quantité d'ultrafiltrat s'effectue en relevant la graduation atteinte par le niveau d'ultrafiltrat et sur la base de cette information, on agit sur la vitesse de la pompe précitée pour réguler la vitesse d'ultrafiltration.

Il est évident qu'un système de contrôle du type indiqué ci-dessus peut être source d'erreur dans la lecture des graduations du récipient et surtout ne permet pas de contrôler de façon automatique le processus d'ultrafiltration.

Le but de la présente invention est de proposer un dispositif de mesure de la quantité d'ultrafiltrat qui permette de surmonter les inconvénients présentés par les systèmes de mesure de types connus mentionnés ci-dessus.

Ce but peut être atteint par la présente invention grâce à un dispositif de mesure de la quantité de liquide extraite d'un circuit de liquide de dialyse, le dispositif comprenant:

une pompe apte à extraire du circuit de liquide de dialyse une quantité de liquide correspondant à la quantité d'ultrafiltrat éliminé du sang,

un premier et un second récipient reliés chacun au moyen de premier et second élément intercepteur de fluide au refoulement de ladite pompe et à un tube d'évacuation,

une première et une seconde sonde de mesure capable chacune de détecter la présence d'ultrafiltrat à un niveau prédéterminé dans le premier et le second récipient et d'émettre en conséquence des signaux électriques, et

des moyens d'élaboration capables d'effectuer le décompte desdits signaux électriques, afin d'établir ladite quantité d'ultrafiltrat éliminé, ainsi que de commander alternativement lesdits éléments intercepteurs de fluide pour obtenir alternativement la liaison dudit premier récipient avec le refoulement de ladite pompe et simultanément la liaison dudit second récipient avec ledit tube d'évacuation, et vice versa.

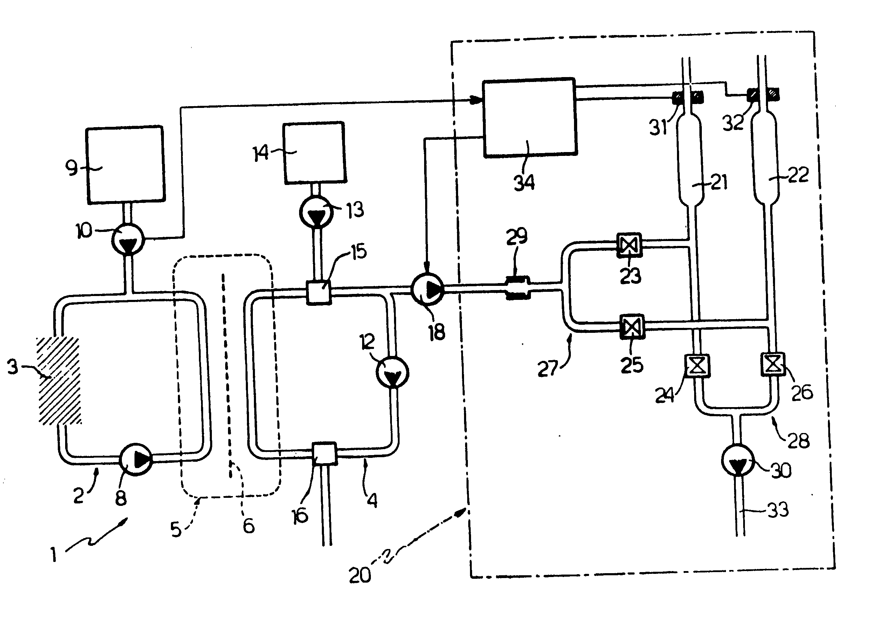

Pour une meilleure compréhension de la présente invention, on va maintenant décrire une forme de réalisation préférée à simple titre d'exemple non limitatif et en référence au dessin ci-joint qui en illustre un schéma avec des blocs de type fonctionnel.

En se référant particulièrement à un tel schéma, 1 désigne dans son ensemble l'appareil de dialyse présentant essentiellement un premier circuit 2 dans lequel le sang est capable de circuler depuis un patient indiqué par la référence 3 et un second circuit 4, dans lequel on fait circuler le liquide de dialyse. Les circuits 2 et 4 traversant un dialyseur 5 présentant un élément 6 (par exemple une membrane de type semi perméable).

Plus particulièrement, le circuit 2 est muni d'une pompe 8 (par exemple du tpye à rouleaux), capable de faire circuler le sang et pouvant recevoir du liquide "perfusé" contenant diverses substances, pendant le traitement de dialyse. Le liquide de perfusion est contenu dans un récipient convenable 9 qui communique avec le circuit 2 au moyen d'une pompe de perfusion 10.

Le circuit 4 présente aussi une pompe 12 capable de faire circuler le liquide de dialyse, le circuit 4 étant alimenté lui-même par une pompe 13 qui prélève le liquide de dialyse depuis un récipient convenable 14 et l'envoie dans le circuit 4 au moyen d'un dispositif distributeur 15. Un tel circuit 4 est également capable de décharger à l'extérieur le liquide de dialyse au moyen d'un dispositif évacuateur 16. En outre le circuit 4 est mis en communication avec l'aspiration d'une pompe 18 dont le but est de créer une dépression dans le circuit 4, afin d'obtenir l'ultrafiltration du sang. Evidemment le circuit 4 est construit dans un matériau de type essentiellement non déformable élastiquement.

Selon la présente invention, la mesure de l'ultrafiltrat qui est éliminé au moyen de la pompe 18, est effectuée grâce à un dispositif indiqué dans son ensemble par la référence 20. Celui-ci comprend essentiellement deux récipients 21, 22, constitués essentiellement par 2 réservoirs cylindriques qui communiquent chacun respectivement avec le refoulement de la pompe 18 et avec un tube d'évacuation 33 au moyen de deux électrovalves respectivement 23, 24 (en ce qui concerne le récipient 21,) et 25, 26 (en ce qui concerne le récipient 22). Plus particulièrement à l'aval des récipients 21 et 22 sont disposées les extrémités des deux tubes en Y, 27 et 28, en forme de fourche dont les parties communes communiquent respectivement avec le refoulement de la pompe 18 au moyen d'un joint d'accouplement 29 et, respectivement, avec le tube 33 au moyen d'une pompe 30.

Chacun des récipients 21, 22 a, en correspondance avec un niveau prédéterminée, une sonde de mesure respectivement 31, 32 (par exemple du type à ultrasons ou du type optique) capable d'indiquer la présence d'ultrafiltrat et d'émettre en conséquence un signal électrique. Le terminal d'une telle sonde est relié à l'entrée d'un circuit d'élaboration 34, lequel, d'une manière qui n'est pas représentée, comprend un compteur convenable, capable de compter le nombre de signaux électriques reçus des sondes 31, 32 pour pouvoir établir, comme illustré par la suite, la quantité d'ultrafiltrat éliminé au moyen de la pompe 18. Le circuit d'élaboration 34 reçoit des signaux de la pompe 10 relatifs à la quantité de liquide perfusé et émet en outre des signaux de la commande de la pompe 18 (ultrafiltrat), ainsi que, d'une manière non représentée, des signaux de commande de la position des électrovalves 23, 24, 25, 26. En particulier, l'électrovalve 23 est commandée, de l'ouverture à la fermeture, en parallèle à l'électrovalve 26. De la même manière, on commande en parallèle les électrovalves 24 et 25. Ainsi, pendant que l'un des 2 récipients 21, 22 se remplit, l'autre se vide, et vice versa.

En usage, on met en mémoire dans le circuit 34 la perte de poids que le patient devra subir pendant le traitement de dialyse. Ensuite on commence le traitement au cours duquel la pompe 10 fournit de façon continue au circuit 34 les données sur les quantités perfusées. Ce circuit 34 régule alors l'ultrafiltration en agissant sur la pompe 18, sur la base des informations reçues par les sondes de mesure 31, 32. Plus particulièrement dans le circuit 4 on fiat circuler le liquide de dialyse au moyen de la pompe 12, liquide qui est périodiquement remplacé grâce aux dispositifs commutateurs 15 et 16 et en actionnant la pompe 13. En outre, la pompe 18 exerce une dépression dans le circuit 4 qui crée les conditions pour obtenir l'ultrafiltration. L'ultrafiltrat qui est éliminé par la pompe 18, est acheminé, par exemple d'abord dans le récipient 21 en tenant fermé l'electrovalve 25 et ouvertes les électrovalves 23 et 26. De cette manière, le niveau de l'ultrafiltrat à l'intérieur du récipient 21 s'élève graduellement jusqu'à atteindre le niveau de la sonde de mesure 31. D'une manière correspondante, l'ouverture de la valve 26 permet à l'ultrafiltrat contenu dans le récipient 22 de s'écouler vers l'évacuation, au moyen de la pompe 30. Quand le niveau de l'ultrafiltrat à l'intérieur du récipient 21 atteint le niveau de la sonde de mesure 31, celle-ci envoie un signal électrique au circuit d'élaboration 34, lequel décompte ces signaux électriques et inverse les signaux de commande des électrovalves 23, 24, 25, 26 de façon à décharger l'ultrafiltrat contenu dans le récipient 21 et à permettre le remplissage du récipient 22, jusqu'à ce que soit atteint le niveau de la sonde de mesure 32. Evidemment, le signal émis par celle-ci est enregistré par le circuit 34, ce qui entraine une nouvelle commutation des commandes des électrovalves précitées.

Dans le cas où un défaut empêcherait le fonctionnement normal du dispositif 20, il serait possible de le déconnecter de la pompe 18, en agissant simplement sur le joint d'accouplement 29, de façon à relier l'évacuation de la pompe 18 elle-même à un récipient gradué de type traditionnel.

Au vu des caractéristiques du dispositif 20 réalisé selon la présente invention on voit les avantages évidents que l'on peut obtenir. Avant tout la mesure de la quantité d'ultrafiltrat offre un degré élevé de précision (dépendant essentiellement du volume des récipients 21, 22) et avant tout, elle n'est pas influencée par l'aspect subjectif de lectures. En outre, il est possible d'obtenir avec précision une diminution du poids du patient à tout moment du traitement de dialyse, étant donné que l'on connait à tout moment le volume du liquide perfusé au patient dans le circuit 2 et le volume d'ultrafiltrat éliminé du circuit 4.

Il résulte enfin clairement qu'au dispositif 20 décrit ci-dessus peuvent être apportées des modifications et variantes sans pour autant sortir de la présente invention.

## Revendications

1. Dispositif de mesure de la quantité de liquide extraite d'un circuit de liquide de dialyse, le dispositif comprenant:

une pompe 18 apte à extraire du circuit de liquide de dialyse une quantité de liquide correspondant à la quantité d'ultrafiltrat éliminé du sang,

un premier et un second récipient 21, 22 reliés chacun au moyen de premier et second élément intercepteur de fluide 23, 24, 25, 26 au refoulement de ladite pompe 18 et à un tube d'évacuation 33,

une première et une seconde sonde de mesure 31, 32, capable chacune de détecter la présence d'ultrafiltrat à un niveau prédéterminé dans le premier et le second récipient 21, 22 et d'émettre en conséquence des signaux électriques, et

des moyens d'élaboration 34 capables d'effectuer le décompte desdits signaux électriques, afin d'établir ladite quantité d'ultrafiltrat éliminé, ainsi que de commander alternativement lesdits éléments intercepteurs de fluide 23, 24, 25, 26 pour obtenir alternativement la liaison dudit premier récipient 21 avec le refoulement de ladite pompe 18 et simultanément la liaison dudit second récipient 22 avec ledit tube d'évacuation 33, et vice versa.

2. Dispositif selon la revendication 1, caractérisé en ce que entre lesdits éléments intercepteurs de fluide 23, 25 et ladite pompe 18, on interpose un dispositif de connection 29 qui permet le désaccouplement entre ladite pompe 18 et lesdits éléments intercepteurs 23, 25.

3. Dispositif selon l'une des revendications 1 ou 2, caractérisé par le fait qu'il comprend une pompe auxiliaire 30 disposée entre lesdits éléments intercepteurs 24, 26 et ledit tube d'évacuation 33.

4. Dispositif selon l'une quelconque des revendications précédentes, caractérisé par le fait que:

lesdites sondes de mesure 31, 32 sont, soit du type à ultrasons, soit du type optique.

5. Dispositif selon l'une quelconque des revendications précédentes caractérisé par le fait que:

lesdits éléments intercepteurs de fluide 23, 24, 25, 26 sont essentiellement constitués d'électrovalves pilotées par lesdits moyens d'élaboration 34.

6. Dispositif selon l'une quelconque des revendications précédentes, caractérisé par le fait que:

lesdits moyens d'élaboration 34 sont capables d'envoyer un signal pilote à ladite pompe 18.

**Patentansprüche**

1. Vorrichtung zum Messen der Flüssigkeitsmenge, die aus einem Dialyseflüssigkeitskreislauf entnommen wurde, wobei die Vorrichtung umfaßt:

eine Pumpe (18), die geeginet ist, dem Dialyseflüssigkeitskreislauf eine Flüssigkeitsmenge zu entziehen, die der Menge des Ultrafiltrats, das dem Blut entnommen wurde, entspricht,

einen ersten und einen zweiten Behälter (21, 22), wobei jeder durch ein erstes und zweites Fluidunterbrechungselement (23, 24, 25, 26) mit der Druckseite der Pumpe (18) und einer Abflußleitung (33) verbunden ist,

eine erste und eine zweite Meßsonde (31, 32), wobei jede Meßsonde das Vorhandensein von Ultrafiltrat auf einem vorbestimmten Niveau in dem ersten und zweiten Behälter (21, 22) feststellen und daraufhin elektrische Signale aussenden kann, und

Verarbeitseinrichtungen (34), die die elektrischen Signale verarbeiten können, um die Menge des entnommenen Ultrafiltrats festzustellen und um abwechselnd die Fluidunterbrechungselemente (23, 24, 25, 26) zu steuern, um abwechselnd die Verbindung des ersten Behälters (21) mit der Druckseite der Pumpe (18) zu erreichen und gleichzeitig die Verbindung des zweiten Behälters (22) mit der Abflußleitung (33) und umgekehrt.

2. Vorrichtung nach Anspruch 1, dadurch gekennzeichnet, daß zwischen den Fluidunterbrechungselementen (23, 25) und der Pumpe (18) eine Verbindungsvorrichtung (29) angeordnet wird, die das Loskoppeln der Pumpe (18) von den Fluidunterbrechungselementen (23, 25) erlaubt.

3. Vorrichtung nach einem der Ansprüche 1 oder 2, dadurch gekennzeichnet, daß sie eine Hilfspumpe (30) aufweist, die zwischen den Unterbrechungselementen (24, 26) und der Abflußleitung (33) angeordnet ist.

4. Vorrichtung nach einem der vorhergehenden Ansprüche, dadurch gekennzeichnet, daß die Meßsonden (31, 32) Ultraschall- oder optische Sonden sind.

5. Vorrichtung nach einem der vorhergehenden Ansprüche, dadurch gekennzeichnet, daß die Fluidunterbrechungselemente (23, 24, 25, 26) im wesentlichen Elektroventile sind, die von den Verarbeitungseinrichtungen (34) gesteuert werden.

6. Vorrichtung nach einem der vorhergehenden Ansprüche, dadurch gekennzeichnet, daß die Verarbeitungseinrichtungen (34) ein Steuersignal an die Pumpe (18) senden können.

**Claims**

1. A device for measuring the quantity of liquid extracted from a dialysis liquid circuit, the device comprising:

a pump (18) capable of extracting from the dialysis liquid circuit a quantity of liquid corresponding to the quantity of ultrafiltrate eliminated from the blood.

a first and a second vessel (21, 22) each connected by means of a first and second fluid interceptor element (23, 24, 25, 26) to the delivery side of the said pump (18) and to a discharge tube (33),

a first and a second measuring sensor (31, 32) each capable of detecting the presence of ultrafiltrate at a predetermined level in the first and second vessel (21, 22) and of emitting electric signals as a result, and

processing means (34) capable of analysing the said electric signals so as to establish the said quantity of ultrafiltrate eliminated, as well as of alternately actuating the said fluid interceptor elements (23, 24, 25, 26) to obtain alternately the connection of the said first vessel (21) to the delivery side of the said pump (18) and simultaneously, the connection of the second vessel (22) to the said discharge tube (33) and vice versa.

2. A device according to Claim 1, characterized in that between the said fluid interceptor elements (23, 25) and the said pump (18), there is interposed a connecting device (29) allowing the said pump (18) and the said interceptor elements (23, 25) to be disconnected from each other.

3. A device according to one of Claims 1 or 2, characterized in that it comprises an auxiliary pump (30) disposed between the said interceptor elements (24, 26) and the said discharge tube (33).

4. A device according to any one of the preceding Claims, characterized in that:

the said measuring sensors (31, 32) are either of the ultrasonic or optical type.

5. A device according to any one of the preceding Claims, characterized in that:

the said fluid interceptor elements (23, 24, 25, 26) are mainly constituted by electrovalves controlled by the said processing means (34).

6. A device according to any one of the preceding Claims, characterized in that:

the said processing means (34) are capable of transmitting an actuating signal to the said pump (18).